# EUROPEAN PATENT APPLICATION

(11) **EP 2 567 684 A1**
(43) Date of publication of application: **13.03.2013**
(21) Application number: 12183034.3
(22) Date of filing: 05.09.2012
(51) Int. Cl.: A61F 13/44, A61F 13/84, A61F 13/00

(54) **A radiologically visible non-woven fabric gauze**

(30) Priority: 06.09.2011 ES 201131458
(71) Applicant: BC Nonwovens, S.L., 08036 Barcelona (ES)
(72) Inventor: Vinas Pich, Carlos, ES-08036 Barcelona (ES)
(74) Representative: Barroso Sánchez-Lafuente, Ignacio M.

(57) **Abstract**

A gauze being formed in a sheet (1) of non-woven fabric comprising first fibres (2) of radiologically transparent or invisible material and second fibres (2) of radiologically visible or opaque material mixed and distributed throughout the whole extension of the sheet (1), in a by-weight proportion being sufficient to assure that any portion of the sheet (1) of non-woven fabric will be radiologically visible.

## Description

### Object of the invention

The present invention relates to a radiologically visible non-woven fabric gauze that can be detected in a surgical intervention by means of a radiography before closing the intervention in order to prevent the abandonment of said gauze inside the patient.

### Application field of the invention

This gauze is applicable in the sanitary material industry, and more concretely in the sector of the manufacture of sterile dressings and gauzes for surgical interventions.

### Background of the invention

In the course of a surgical operation or in the healing of a deep wound it is common to use sterile gauzes introduced in the operation region, or inside the wound, in order to soak them with the blood and to thus facilitate the task of the sanitary personnel and the physicians.

Before finishing the intervention all those gauzes must be removed from the patient in order to prevent ulterior problems with infections and health risks. These gauzes soaked with blood are nevertheless difficult to distinguish from the surrounding tissues and organs, and sometimes the soaked gauze is torn and thus leaves a torn piece that can pass unnoticed.

Techniques are already known which aim at making the fabric gauzes generally having a warp and weft structure radiologically visible and at making it possible to detect them in a radiography taken from the patient before suturing the wound or the intervention.

For example in the Spanish patent ES497130 for "A process for the obtainment of a radiologically opaque yarn" the existence in the market of yarns coated with X-ray opaque matters is already considered as known, but with the drawback of the impossibility to have them woven into the weft of the gauze since the coating determines a rough and easily strippable surface coat.

Said patent also specifies a technique having been used before as based on this problem and consisting in superimposing on the outer surface of the fabric forming the gauze the aforementioned yarns coated with X-ray opaque matters. This fixation to the outside of the gauze is carried out by means of pressure and heat fixation or by means of an adhesive strip, the yarn in question running a risk of being stripped off if this fixation is not properly carried out. This fixation is besides slow and slows down the production of the gauze.

The solution proposed in the aforementioned Spanish patent ES497130 consists in a process for the obtainment of a radiologically opaque yarn that is in a position to be woven with conventional textile machines and to be incorporated into the weft or warp of the gauze thus precluding the possibility of its being stripped off and providing some reliability in the scanning with X-rays.

An also known document is the Spanish utility model ES1045580U for "A gauze for surgical interventions" where a gauze for surgical interventions is described which is made up of an actual gauze of any conventional type incorporating at least a metallic filament being detectable through electromagnetic means and/or through X-rays.

This document provides for the filament in question to be properly fixed to the base body of the gauze, such as for example by being interlocked in the network of this latter after the obtainment of said base body. The radiologically visible yarn is fixed by means of sewing, an adhesive strip, heat welding or any other conventional locking means within the general context of the gauze. The main problem with this technique is the reduced manufacturing speed. The radiologically visible yarn can besides be stripped off from the surface of the base body if the fixation has not been carried out in a completely adequate manner, the gauze being thus deprived from the feature of radiologic detection.

The European patent EP0272901 for "A surgical fabric with a printed X-ray marker" (Johnson & Johnson Medical) having been validated in Spain with the publication number ES2035868 relates to a method for the preparation of a surgical sponge that comprises an absorbing fabric with an X-ray detectable marker associated to it, said method consisting in applying a fluid radio-opaque polymer composition to the surface of the aforementioned fabric thus forming a visually distinctive pattern, and solidly adhering said polymer composition to said fabric.

The Spanish patent document ES494396 for "A process for obtaining a Roentgen-ray opaque yarn" (Juan Vilanova) is known which aims at incorporating a radiologically opaque yarn in a variable number into the weft or warp of a cotton fabric being used to form the gauzes for clinical use. This process is essentially **characterised in that** it consists in incorporating barium into a volume of viscose in a proportion of between 45 and 75 %, said blend being then submitted to extrusion and ulterior twisting in order to thus form the yarn.

In none of the aforementioned cases the gauzes allow that if a small torn off portion of the gauze is left behind this portion will still be detectable. In interventions of a certain type it is in effect common for said gauzes to be torn apart when they are deeply soaked with blood, and if the portion remaining inside the wound or intervention region does not have the radiologically visible yarn it is then undetectable.

### Description of the invention

The radiologically visible non-woven fabric gauze being the object of this invention has technical particularities that allow for an optimum and safe operation at all times and thus allow for an X-ray detection of even those portions possibly being separated from the gauze due to an accidental tearing of this latter during the intervention.

The gauze is of the type of those being formed in a mono- or multilayer sheet of non-woven fabric.

According to the invention the gauze comprises first fibres of radiologically transparent or invisible material and second fibres of radiologically visible or opaque material mixed with the first fibres and distributed throughout the whole extension of the sheet in a by-weight proportion being sufficient to assure that any portion of the sheet of non-woven fabric will be radiologically visible.

The manufacture of the gauze of the invention is simple and cost effective without having to excessively alter already known processes of manufacture of non-woven fabrics. This process of manufacture of the gauze of the invention contrasts with other processes of manufacture of gauze fabric with a warp and weft structure or by means of an external application of radiologically visible yarns or by means of using radiologically visible substances with which to impregnate the conventional gauzes in ulterior treatments.

The distribution of the radiologically visible fibres throughout the whole extension of the gauze contributes to allow any portion of the gauze possibly having been torn off by accident during its manipulation to be detected with X-rays and thus prevents that the torn off portion can pass unnoticed or be undetected, such as in the case of the gauzes that are provided with a radiologically visible yarn adhered in a localised region of their surface.

In one embodiment the second fibres of radiologically visible material make up a range of 3 to 70 percent of the total weight of fibres forming the gauze, and preferably a range of 5 to 20 percent.

In one embodiment the second fibres of radiologically visible material have a length of 20 to 60 millimetres and a diameter of 5 to 30 micrometres. Within this range of dimensions said second fibres preferentially have a length of 40 millimetres and a diameter of 21 to 23 micrometres and thus allow to obtain a good adhesion of the fibres to each other when forming the sheet of non-woven fabric that makes up the gauze.

In one embodiment the second fibres of radiologically visible material comprise in their formulation between 30% and 80% by weight of barium sulphate. In a preferential case the second fibres of radiologically visible material comprise viscose as base material used to integrate the barium sulphate, although not precluding the use of other substances allowing to compound the barium sulphate into the composition of the fibres before spinning or extruding them.

### Description of the figures

In order to complement the description being made and for the purpose of facilitating the understanding of the characterising features of the invention the present specification is being accompanied by a set of drawings wherein the following has been represented with an illustrative and nonlimiting character:

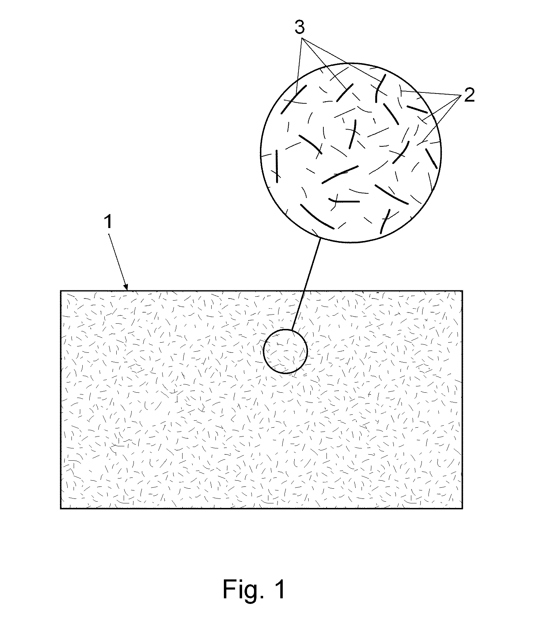

- Figure 1 shows a plan view of a gauze with an enlarged detail view of the fibre distribution.

### Preferential embodiment of the invention

As can be seen in the abovementioned figures the gauze is formed by a sheet (1) of non-woven fabric comprising first fibres (2) of radiologically transparent material and second fibres (3) of radiologically visible material, the second fibres (3) being distributed throughout the whole sheet (1) of non-woven fabric forming the gauze.

In this case the first fibres (2) of radiologically transparent material comprise fibres of viscose and polyester in a variable proportion to each other, and approximately 10 percent of the total weight of the sheet (1) of non-woven fabric is made up by the second fibres (3) of radiologically visible material. In this case the second fibres (3) are made up by viscose that is compounded with barium sulphate before spinning said second fibres in order to incorporate the barium sulphate into the fibre, said barium sulphate making up 60 percent of the total weight of said second fibres (3) of radiologically visible material.

The fibres (2, 3) have an approximate length of 40 millimetres and a diameter of 21 micrometres for fibre (3) and 12 micrometres for fibre (2).

Once having sufficiently described the nature of the invention, as well as a preferential exemplary embodiment, it is hereby stated for the record and for all relevant purposes that the materials, shape, size and arrangement of the described elements can be modified, provided that this does not involve an alteration of the essential features of the invention which are claimed below.

## Claims

1. A radiologically visible non-woven fabric gauze of the type of those being formed in a mono- or multilayer sheet (1) of non-woven fabric, **characterised in that** it comprises first fibres (2) of radiologically transparent material and second fibres (3) of radiologically visible material mixed with the first fibres (2) and distributed throughout the whole extension of the sheet (1), in a by-weight proportion being sufficient to assure that any portion of the sheet (1) of non-woven fabric will be radiologically visible.

2. A gauze as per claim 1, **characterised in that** the second fibres (3) of radiologically visible material make up a range of 3 to 70 percent of the total weight of fibres in the sheet (1).

3. A gauze as per claim 2, **characterised in that** the second fibres (3) of radiologically visible material approximately make up a range of 5 to 20 percent of the total weight of fibres in the sheet (1).

4. A gauze as per any of the foregoing claims, **characterised in that** the second fibres (3) of radiologically visible material have a length of 20 to 60 millimetres and a diameter of 5 to 50 micrometres.

5. A gauze as per claim 4, **characterised in that** the second fibres (3) of radiologically visible material have a length of 40 millimetres and a diameter of 21 to 23 micrometres.

6. A gauze as per any of the foregoing claims, **characterised in that** the second fibres (3) of radiologically visible material comprise in their formulation from 30 to 80 percent by weight of barium sulphate.

7. A gauze as per claim 6, **characterised in that** the second fibres (3) comprise viscose.
